# EUROPEAN PATENT SPECIFICATION

(11) **EP 2 651 518 B1**
(45) Date of publication and mention of the grant of the patent: **26.06.2019**
(21) Application number: 10793407.7
(22) Date of filing: 13.12.2010
(51) Int. Cl.: A61Q 11/00, A61K 8/73, A61K 8/92, A61K 8/34

(54) **ORAL COMPOSITIONS AND METHOD FOR PRODUCING THEREOF**
ORALE ZUSAMMENSETZUNGEN UND HERSTELLUNGSVERFAHREN DAFÜR
COMPOSITIONS ORALES ET PROCÉDÉ POUR PRODUIRE CELLES-CI

(43) Date of publication of application: 23.10.2013
(73) Proprietor: Colgate-Palmolive Company, New York, NY 10022 (US)
(72) Inventor: GU, Ben, East Brunswick, New Jersey 08816 (US); HASSAN, Mahmoud, Somerset, New Jersey 08873 (US)
(74) Representative: Wibbelmann, Jobst
(86) International application number: PCT/US2010/060103
(87) International publication number: WO 2012/082101

(56) References cited:
- US-A1- 2005 019 273
- US-A1- 2008 014 224
- US-B1- 6 669 929

## Description

### FIELD OF THE INVENTION

This invention relates to methods of making dentifrice products comprising hydratable, polymer matrix films.

### BACKGROUND OF THE INVENTION

The hydratable, polymer matrix films which are produced free of low solubility flavorants are combined with dentifrice base that comprises low solubility flavorant which migrates from the dentifrice base into the hydratable, polymer matrix films to form a dentifrice that comprises hydratable, polymer matrix films which include low solubility flavorants.

Liquid, gel and semi-solid oral care products which comprise hydratable, polymer matrix films containing low solubility flavorants such as menthol are known. Hydratable, polymer matrix film containing low solubility flavorants such as menthol are prepared and added into toothpaste to generate an aesthetic effect as well as to provide the benefit of a flavor and/or cooling sensation or signal. The hydratable, polymer matrix film, typically in the form of flakes or small sized pieces cut from larger manufactured films, is maintained in the product when stored. Upon use, the films typically degrade by chemical or physical disruption, thereby releasing the active or functional material into the surrounding environment. In this manner, the films provide an opportunity for localized release of a high concentration of active materials, such as for example zinc oxide, near a target surface. In addition, low solubility flavorant in the film is also released. The low solubility flavorants such as menthol in the films provide an extended flavor experience to the user compared to that which occurs when compositions in which the flavor is only in the toothpaste base are used. By including flavorants in the films, flavor may be released from films during and immediately after use, providing a flavor experience that continues after performance of the oral care process such as brushing or rinsing is completed. This extended experience can be pleasing.

Conventional methods of manufacturing hydratable, polymer matrix films that contain low solubility flavorants such as menthol comprise the step of incorporating menthol into the slurry that is then used to form the film. The low solubility flavorant is thereby dispersed throughout the slurry which is used to make the film. After the film is formed, it is typically often cut into flakes or pieces, and introduced into the toothpaste base. The step of adding relatively insoluble flavorant into the slurry used to manufacture the hydratable, polymer matrix films typically requires the use of solvents such as ethanol. When making the films, the ethanol is typically removed using heat which causes the ethanol to evaporate. The low solubility flavorants are lost as a result of evaporation of the ethanol solvent. For examples, about 50% of menthol in a slurry formula is lost with the solvent when the ethanol evaporates. Moreover, the evaporated ethanol creates a safety concern in the manufacturing facility. Accordingly, introduction of low solubility flavorants into the film during its manufacture is inefficient, which leads to in additional manufacturing costs, and creates conditions which must be managed to avoid safety problems.
US 2008/0014224 A1 discloses methods of making films having active ingredients. The films can be used in a variety of applications, including oral care compositions.

There is a need for improved methods of manufacturing liquid, gel and semi-solid oral care products which comprise hydrophilic films containing menthol.

### BRIEF SUMMARY OF THE INVENTION

The present invention concerns a method of preparing a dentifrice and a dentifrice product as defined in independent claims 1 to 7. Further embodiments are set forth in the claims dependent on said independent claims.

### DETAILED DESCRIPTION OF THE INVENTION

### Definitions

As used herein, the term "cellulose polymer" is meant to refer to cellulose and cellulose derivatives such as cellulose ester derivative and cellulose ether derivatives.

As used herein, the term "dentifrice" includes toothpastes and gels.

As used herein, such a "pharmaceutically acceptable" or "cosmetically acceptable" component is one that is suitable for use with humans and/or animals to provide the desired therapeutic, prophylactic, sensory, decorative, or cosmetic benefit without undue adverse side effects (such as toxicity, irritation, and allergic response) commensurate with a reasonable benefit/risk ratio.

As used herein, the term "polymer matrix film" is meant to refer to the product of a process wherein cellulose and derivatives thereof are used in combination with other polymers to form thin solid water hydratable film which may further comprise other components including, colloids and other particles. The polymer matrix film for example may further comprise additives such as, for example, colorants, water soluble flavorants, sweeteners, breath fresheners, whitening agents, and/or therapeutic agents such as agents that promote oral health, e.g. healthy teeth, gums and other oral tissue, and agents that prevent and treat various oral maladies. In addition, the polymer matrix film may include other film forming agents, plasticizing agents, surfactants and emulsifying agents. The polymer matrix film may be cut or otherwise divided into multiple pieces such as flakes or small strips and added to a dentifrice where they may provide aesthetic elements and/or serve as a carrier for one or more additives which may be included.

As used herein, the term "low solubility flavorant" refers to a flavor ingredient or cooling agent which is relatively insoluble in water, i.e. having the solubility generally on the order of menthol in water or less soluble. A "low solubility flavorant" must first be incorporated into a solution using a solvent such as an alcohol, particularly ethanol, in order to stably incorporate it into the slurry of hydrophilic hydratable polymer which can be used to produce hydratable polymer matrix films comprising low solubility flavorants.

As used herein, the term "low solubility flavorant-free polymer matrix film" is meant to refer to a polymer matrix film that is in the substantial absence of low solubility flavorant. Low solubility flavorant-free polymer matrix films are produced without the direct addition of low solubility flavorant, or of ingredients or solutions containing low solubility flavorant into the slurry used to make the low solubility flavorant-free polymer matrix film.

As used herein, the term "substantial absence" is meant to refer to a film that has a low solubility flavorant content on film formation of less than 0.5% .

As used herein, the term "transferring" refers to migration, moving or transporting flavorant from the dentifrice base into the film. Passive transfer typically does not require an external agent (e.g., mechanical force, chemical and/or thermal energy) to achieve movement of the low solubility flavorant. Passive transfer typically encompasses mass transport phenomena including diffusion, where the flavorant molecules are physically transported across a concentration gradient to approach thermodynamic equilibrium. Further, passive transfer may include electrochemical interaction, absorption, adsorption, and/or wicking movement of the flavorant into the film, where application of an external agent is not required to achieve sufficient movement of the flavorant into the film. Active transport is generally not required. However, in some embodiments, ingredients may be provided to drive equilibrium to promote transfer of flavorant from the dentifrice base to the polymer matrix film.

Throughout the present disclosure, ranges are used as shorthand for describing each and every value that is within the range. Any value within the range can be selected as the terminus of the range. Furthermore, all references cited throughout the disclosure are expressly incorporated by reference in their entireties. As used herein, all references to concentration of ingredients are on a weight basis, unless otherwise indicated.

Unless otherwise specified, all percentages and amounts expressed herein and elsewhere in the specification should be understood to refer to percentages by weight. The amounts given are based on the active weight of the material.

### Overview

Aspects of the present invention relate to methods of making a gel and semi-solid oral care products such as toothpaste which comprise polymer matrix films that contain low solubility flavorant such as menthol. Some of the methods comprise producing a low solubility flavorant-free hydratable polymer matrix and eliminating the use of alcohol that is required when incorporating low solubility flavorant into the polymer slurry used to make polymer matrix films. The low solubility flavorant-free hydratable polymer matrix film is combined with a dentifrice base that comprises a low solubility flavorant and the low solubility flavorant transfers from the dentifrice base into the polymer matrix film. Thus, after being combined with the dentifrice base that comprises low solubility flavorant, flavorant becomes incorporated into what was formerly low solubility flavorant-free polymer matrix film and the resulting combination includes polymer matrix films that comprise low solubility flavorant dispersed in dentifrice base. The low solubility flavorant is transferred from dentifrice base into the polymer matrix film such that the polymer matrix film contains flavorant at a concentration nearly equal to, equal to or greater than the concentration of flavorant in the base. The transferred flavorant is stable during storage of the composition. The transferred flavorant is stable during storage of the composition.

In various embodiments, the invention provides methods which eliminate a step performed in the conventional process of making dentifrice products that comprise polymer matrix films which include menthol as low solubility flavorant. The step that may be eliminated is a step involving making a flavorant solution having low solubility flavorant in a solvent such as ethanol or another alcohol or solvent system. In the conventional process, the flavorant solution is included in the slurry that is processed into the polymer matrix film. The alcohol used to incorporate the low solubility flavorant into the slurry is removed by evaporation. By removing the alcohol in this way, low solubility flavorant is lost from the slurry with the alcohol in the evaporation process, resulting in the actual amount of low solubility flavorant in the polymer matrix films to be less than the amount of low solubility flavorant added to the slurry. This loss increases costs and the need for larger amounts of low solubility flavorant. Moreover, steps must be taken to prevent any hazards that may arise in the manufacturing process due to evaporated alcohol in the manufacturing facility.

Thus, in the conventional method, the polymer matrix films contain menthol as low solubility flavorant prior to their addition to the dentifrice base. The preparation and use of an alcohol-based solution is eliminated by producing low solubility flavorant-free polymer matrix films and combining them into dentifrice base that comprises a solubility flavorant. When low solubility flavorant-free polymer matrix films are included in a dentifrice base that comprises low solubility flavorant, the flavorant migrates from the base into the polymer matrix films. The resulting product is a dentifrice having hydratable, polymer matrix films which comprise low solubility flavorant. The modification of the conventional process reduces costs of materials and eliminates potential safety issues that exist when using ethanol solutions.

### Polymer matrix films

Polymer matrix films provided herein comprise hydroxyl propyl methyl cellulose as a water soluble polymer.

Polymer matrix films may also comprise numerous other ingredients.

Typically, polymer matrix films comprise polymers present in an amount between 30% and 90% of the polymer matrix film's dry weight. The polymers may be present in an amount of between 40% and 80% of the polymer matrix film's dry weight. Some embodiments comprise polymers in an amount between 40% and 70% of the polymer matrix film's dry weight. Some embodiments comprise polymers an amount between 40% and 60% of the polymer matrix film's dry weight. Some embodiments comprise polymers an amount between 40% and 50% of the polymer matrix film's dry weight. Some embodiments comprise polymers in an amount between 50% and 80% of the polymer matrix film's dry weight. Some embodiments comprise polymers an amount between 60% and 80% of the polymer matrix film's dry weight. Some embodiments comprise polymers an amount between 65% and 75% of the polymer matrix film's dry weight.

Films useful for the present invention may be rigid or flexible, comprising any of a variety of materials, including film forming materials. In some embodiments, the film comprises at least one film-forming material, preferably comprising a polymer.

The polymer matrix film is hydratable and is free of low solubility flavorants. Additionally, the formulation of the polymer matrix films may be selected to affect release of active ingredient such as the amount released proportional to how vigorously or how long the composition is used, e.g., by brushing, scrubbing, or other mechanical action during use of the aqueous composition. The formulation of the polymer matrix films may be selected to produce an overall delayed and/or extended release of flavorant, thereby providing a flavor experience following product use.

### Polymers

According to the invention, hydroxypropyl methyl cellulose (HPMC) is used. A family of HPMC products are available commercially from the Dow Chemical Company under the trade designation Methocel™. HPMC products of the Methocel™ family are referred to with the suffix E, F, J or K..

The number following the letter suffix refers to viscosity in millipascal - seconds (mPa-s) measured at a 2% concentration in water at 20°C. A "C" after the number refer to "hundred"; an "M" refers to "thousand". Suffix thereafter are additional identifiers, e.g. "P"' refers to "Premium", "LV"' refers to "Low Viscosity", etc. In some embodiments, one or more Methocel™ products, or generic versions thereof, may be used.

In some embodiments, the film forming agent used to prepare the hydratable polymer matrix films is a low viscosity HPMC. When HPMC is used as the film forming agent, it is preferred that the HPMC have a viscosity in the range of about 1 to about 40 millipascal seconds (mPas) as determined as a 2% by weight aqueous solution of the HPMC at 20° C. using a Ubbelohde tube viscometer. In some embodiments, the HPMC has a viscosity of about 3 to about 20 mPas at 20° C. In some embodiments, the HPMC is Methocel™ E5 LV. Methocel™ E5 LV is a USP grade, low viscosity HPMC having 29.1% methoxyl groups and 9% hydroxyproxyl group substitution. It is a white or off-white free-flowing dry powder. As a 2 wt. % solution in water as measured with an Ubbelohde tube viscometer, the HPMC solution has a viscosity of 5.1 mPas at 20° C. Other examples of METHOCEL™ HPMC products include METHOCEL™ E5, METHOCEL™ E50, METHOCEL™ E15, and METHOCEL™ K100.

Water soluble cellulose derivatives are typically the primary type of polymer. Other types of polymers, however, may be included.

Other useful polymers may include polyvinylpyrrolidone (PVP), which can have a weight average molecular weight of about 100,000 or more and up to about 1.5 million, vinyl acetate, polyvinylpyrrolidone-vinyl acetate copolymers such as KOLLIDON™ VA64 (available from BASF, 60:40 by weight vinyl pyrrolidone) and PLASDONE™ S630 PVP (available from International Specialty Products, Wayne, N.J., United States of America, 60:40 by weight vinyl pyrrolidone:vinyl acetate), ethylene oxide graft copolymers of PVA such as KOLLICOAT™ IR (available from BASF, 75% by weight PVA, 25% by weight polyethylene glycol graft, polyvinyl alcohol (PVA), acrylates and polyacrylic acid, including polyacrylate polymer, cross-linked polyacrylate polymer, cross-linked polyacrylic acid (e.g., CARBOPOL™), vinylcaprolactam/sodium acrylate polymers, methacrylates, maleic poly vinylalkyl ether-maleic acid copolymer (e.g., GANTREZ™.), vinyl acetate and crotonic acid copolymers, polyacrylamide, poly(2-acrylamido-2-methylpropane sulfonate), terpolymers of acrylomethyl propyl sulphonic acid/methylacrylate/styrene monomers, phosphonate styrene polymers, polyethylene phosphonate, polybutene phosphonate, polystyrene, polyvinylphosphonates, polyalkylenes, polyalkylene oxides, including polyethylene oxide, i.e. polyethylene glycol, and carboxy vinyl polymer. As appreciated by a skilled artisan, the film may comprise derivatives, copolymers, and further mixtures of such polymers as well.

Useful water-insoluble polymers include polymers soluble in at least one organic solvent; for example, acrylic copolymers (where carboxylic acid functionality has not been neutralized), cross-linked poly(vinyl pyrrolidone), for example KOLLIDON™ CL or CL-M available from BASF, poly(vinyl acetate) (PVAc), certain cellulose derivatives such as cellulose acetate, cellulose nitrate, alkyl cellulose such as ethyl cellulose, butyl cellulose, and isopropyl cellulose, cellulose acetate phthalate, shellac, ethylene-vinyl acetate copolymers, vinyl acetate homopolymer, silicone polymer (e.g., dimethylsilicone), polymethyl methacrylate (PMMA), polymers insoluble in organic solvents, such as cellulose, polyethylene, polypropylene, polyesters, polyurethane and nylon, natural or synthetic rubber, and mixtures thereof. An example of a suitable, film-forming acrylic copolymer is LUVIMER™ 30E, a 30% by weight solution in ethanol of a tert-butyl acrylate/ethyl acrylate/methyacrylic acid copolymer commercially available from BASF (Florham Park, N.J., United States of America). The water-insoluble polymers may be prepared as dispersions (e.g., by emulsion polymerization) and may be stabilized with suitable emulsifiers. One useful PVAc emulsion, for example, is KOLLICOAT™ SR 30D, a 30 weight % dispersion of PVAc in water stabilized with 2.7 weight percent PVP and 0.3% sodium lauryl sulfate. An example of an acrylic copolymer dispersion is KOLLICOAT™ EMM 30D, a 30% by weight aqueous dispersion of an ethyl acrylate: methyl methacrylate copolymer (weight ratio of ethyl acrylate to methyl methacrylate approximately 2 to 1) with a reported average molecular weight of about 800,000, available from BASF.

Other useful polymers or water-soluble fillers include, without limitation, natural gums such as sodium alginate, carrageenan, xanthan gum, gum acacia, Arabic gum, guar gum, pullulan, agar, chitin, chitosan, pectin, karaya gum, zein, hordein, oliadin, locust bean gum, tragacantha and other polysaccharides; starches such as maltodextrin, amylose, high amylose starch, corn starch, potato starch, rice starch, tapioca starch, pea starch, sweet potato starch, barley starch, wheat starch, waxy corn starch, modified starch (e.g., hydroxypropylated high amylose starch), dextrin, levan, elsinan and gluten; and proteins such as collagen, whey protein isolate, casein, milk protein, soy protein, keratin, and gelatin. The film may further include dispersible or swellable fillers such as modified starch, alginate esters, and divalent or multivalent ion salts of alginates.

Optionally, cold water swellable, physically modified and pregelatenized starches may be used as additives that can function as a texture modifier to increase the stiffness of the polymer film matrix. In the preparation of such starch products, the granular starch is cooked in the presence of water and possibly an organic solvent at a temperature not higher than 10°C higher than the gelatinization temperature. The obtained starch is then dried. Pregelatinized corn starch is available commercially. A useful starch is available under the trade designation Cerestar Polar Tex-Instant 12640 from the Cerestar Company. This Cerestar starch is a pregelatenized, stabilized and crosslinked waxy maize starch. It is readily dispersible and swellable in cold water. In its dry form, the starch is a white free flowing powder with an average flake size no greater than 180 micrometers and 85% of the flakes are smaller than 75 micrometers. It has a bulk density of 44 lbs/ft³. The Cerestar starch has excellent cold storage and freeze-thaw stability. It has a rapid hydration rate and can reach extremely high viscosity without cooking. It has a smooth and creamy texture similar to cook-up starches. It also has excellent paste clarity and a bland flavor. The pregelatinized starch may present in the film matrix in an amount ranging from about 5 to about 20% by weight, and in some embodiments in which it is included, about 10 to about 15% by weight. In some embodiments in which starch is included, the cellulose/cellulose derivative to starch ratio (by weight) may vary from about 1:3 to about 4:1 and preferably about 1:1.5 to about 2.5:1.

In an aqueous composition, the relative amounts of water-soluble polymer and water-insoluble and/or partially water-soluble polymer in the film are preferably such that the film is storage-stable in an aqueous composition but disintegrates during use of the composition. In various embodiments, the film includes an amount of water-soluble polymer that is about 50% to 90% weight of the dry film. In some embodiments, the film includes an amount of water-soluble polymer that is about 55% to 85% weight of the dry film. In some embodiments, the film includes an amount of water-soluble polymer that is about 60% to 80% weight of the dry film. In some embodiments, the film includes an amount of water-soluble polymer that is about 65% to 75% weight of the dry film. In addition to, or instead of, the water-soluble polymer(s), in some embodiments the film may include partially water-insoluble or water-swellable polymers in amounts of about 0.1% to about 50% by weight of the film, preferably about 10% to about 20% weight. In various embodiments, a method of stabilizing hydrophilic films in an aqueous carrier environment uses water-soluble and water-insoluble materials in the film that are balanced for stability while stored in the product carrier, but disintegrate upon use to release the active ingredient contained therein. In some embodiments, non-polymer materials such as colloidal metals for example may be included in the films. The polymers may be present in an amount of between 40% and 80% of the polymer matrix film's dry weight. Some embodiments comprise polymers in an amount between 40% and 70% of the polymer matrix film's dry weight. Some embodiments comprise polymers an amount between 40% and 60% of the polymer matrix film's dry weight. Some embodiments comprise polymers an amount between 40% and 50% of the polymer matrix film's dry weight.

### Colloids and Colloidal particles

In some embodiments, polymer matrix films comprise colloids. The colloid may present in an amount between 10% and 60% of the polymer matrix film's dry weight. The colloid may present in an amount between 20% and 50% of the polymer matrix film's dry weight. The colloid may present in an amount between 30% and 50% of the polymer matrix film's dry weight. The colloid may present in an amount between 40% and 50% of the polymer matrix film's dry weight.

Colloids and colloidal particles can be used to stabilize polymer matrices and fine tune its rigidity in order to provide films that are flexible enough to process, yet physically and cosmetically stable. As films are optimized, it is important to identify the parameters that will deliver optimal film performance. These parameters can be determined by quantifying the properties of the film at both the slurry stage and the dry film stage. At the slurry stage, the interactions between the polymers and the other film ingredients, including colloidal particles, form the structure of the film matrix. The viscoelastic properties of the slurry, such as the viscosity and the structural parameter (G'), enable the characterization of structural arrangement within the slurry and the processability of the same. Following processing and drying of the slurry, the bulk film is formed, setting the polymer matrix. Mechanical properties, such as the glass transition temperature, the tensile strength, and the dissolution time can be used to determine the stability of the film. By balancing the microstructural properties, such as the polymer interactions, with the macrostructural properties of the film, such as the mechanical properties, film can be made more cosmetically stable and can be better utilized as a delivery platform for various actives.

In some embodiments, colloidal particles are present in the film in the range of 40-50% dry weight.

Water-insoluble colloidal metal compounds of multivalent metals are preferred. Representative metal oxides suitable for use in the compositions described herein include silicon oxide (SiO2), molybdenum oxide (Mo2O3), aluminum oxide (Al2O3), titanium oxide (TiO), zirconium oxide (ZrO2) and zinc oxide (ZnO).

Particle size may be about 1 to about 1000 nm. Preferably the particles have an average particle size of about 1 µm to about 850 nm, about 50 µm to about 150 nm, about 15 nm to about 500 nm, about 30 nm to about 250 nm and/or about 5 µm to about 100 nm.

In some embodiments, the particles are non-aggregated. By non-aggregated it is meant that the particles are not massed into a cluster having a size greater than about 1 micron, preferably greater than about 950 nm or 850 nm. However, particles may be mixed with aggregated particles and other colloidal particles that have an average particle size of greater than 1 micron if desired. In some embodiments, more than 80% of particles are non-aggregated. In some embodiments, more than 90% of particles are non-aggregated.

In some embodiments, colloidal particles are provided in the dentifrice base. In some embodiments, colloidal particles are provided in the dentifrice base and the polymer matrix film. In some embodiments, colloidal particles are provided in the dentifrice base but not the polymer matrix film.

### Preparation of Film Matrix

In preparing the film matrix, the polymers and any of the optional ingredients, including for example, such as those set forth below as "Other Components", are dissolved or otherwise mixed into a compatible solvent to form a film forming composition. The film forming composition may contain no flavorant and no flavor solvent. The film forming composition is cast on a releasable carrier and dried to form a sheet of film matrix material. In some embodiments, the carrier material has a surface tension which allows the film solution to spread evenly across the intended carrier width without soaking to form a destructive bond between the film carrier substrates. Examples of suitable carrier materials include glass, stainless steel, Teflon and polyethylene-impregnated paper. Drying of the film may be carried out at high temperature using a drying oven, drying terminal, vacuum drier, or any other suitable drying equipment which does not adversely affect the ingredients of which the film is composed.

The slurries that are precursors to the films may be characterized using rheology. In some embodiments, the viscoelastic properties of the film slurry, as quantified using G' as an indicator of the structural character of the polymer-particle network, may be about 220-560. In some embodiments G' is about 223-550. In some embodiments, the structure of the polymer-particle matrix is not weak and the slurry is not essentially liquid-like. In some embodiments, the structure of the polymer-particle matrix is not very rigid thereby not leading to the formation of a very brittle film. In some embodiments, the viscosity profile as a function of shear rate is quantified as a measure of flowability and processability the slurries. In some embodiments, the viscosity profiles are not a semi-dilute solution. The viscosity in poise is measured at 0.3 s-1. In some embodiments, the viscosity (taken at 0.3 s-1) for the various slurries is about 175-475. In some embodiments, the viscosity (taken at 0.3 s-1) for the various slurries is about 183-450.

The films of the present invention preferably have a substantially lamellar structure. A "lamellar" structure has a size in one or two dimensions (e.g., x- or y-dimensions) that is substantially greater than the thickness of the structure in a third dimension (e.g., the z-dimension), and generally includes substantially planar, layered, or lamelliform shapes, for example. In one embodiment, the lamellar structure is substantially planar, having a size in both the x- and y-dimensions that is substantially greater than the z-dimension. In other embodiments, the lamellar structure is non-planar. In one embodiment, a film comprises a substantially continuous surface that can appear as a substantially flat surface, although in some embodiments the film may be deformed. In such embodiments, the film can have any of a number of shapes, including having a smooth, curved surface. Further, the term "film" encompasses both a single structure as well as a plurality of film fragments. In certain embodiments, the film comprises a plurality of fragments independently having a thickness of about 0.1 mils to about 10 mils, preferably about 0.5 mils to 9 mils, and more preferably about 1.2 mils to about 3 mils. In some embodiments, the film thickness range is 2 to 3 microns. A preferred length of the fragments is at least about 0.2 mm.

The dried film is then processed for inclusion in the dentifrice. The film may be cut or punched into small strips or squares. In various embodiments, the film comprises a plurality of fragments or pieces. Such fragments may be of any of a variety of shapes or forms, including semi-solid or solid discrete portions, fragments, particles, flakes, or mixtures thereof. In various embodiments, the film fragments have a recognizable shape. In some embodiments, a film fragment comprises a nonrandom shape. Such shapes include simple geometric shapes such as polygons, elliptical shapes, triangles, quadrilaterals (such as a square, a rectangle, a rhombus), pentagons, hexagons, ovals, circles, or shapes that are representative of figures, animate or inanimate objects, such as stars, hearts, gems, flowers, trees, shamrocks, letters, numbers, animals, characters, diamonds, circles and the like.. The dried film may be cut or punched into shaped flakes having a particle size of 0.01 to 0.50 inches preferably 0.08 to 0.25 inches. Additional stability can be provided to the shapes formed from the dried film, by applying to the film, before shaping into flakes or small strips, a protective barrier overcoat such as a food grade shellac or ethyl cellulose.

Further, the plurality of film fragments may have different compositions, for example having a first plurality of film fragments comprising a first color, and a second plurality of film fragments comprising a second color, where the first and second colors are different from each other. Any permutation of different compositions is contemplated, for example, any number of different active ingredients in the compositions or different film compositions.

### Base Dentifrice Composition

Examples of suitable carriers for oral care compositions are disclosed in U.S. Pat. Nos. 6,669,929 to Boyd et al., 6,379,654 to Gebreselassie et al., and 4,894,220 to Nabi et al.

The dentifrice (toothpaste or gel) is typically water based. As recognized by one of skill in the art, the dentifrice optionally include other materials and mixtures thereof, including for example, such as those set forth below as "Other Components". It is understood that while general attributes of each of the above categories of materials may differ; there may be some common attributes, and any given material may serve multiple purposes within two or more of such categories of materials.

In the preparation of the base dentifrice in accordance with the present invention there is utilized an orally acceptable vehicle, including a water-phase with humectants. Humectants useful herein include polyhydric alcohols such as glycerin, sorbitol, xylitol or low molecular weight PEGs, alkylene glycol such as polyethylene glycol or propylene glycol. In various embodiments, humectants are operable to prevent hardening of paste or gel compositions upon exposure to air. In various embodiments humectants also function as sweeteners. One or more humectants are optionally present in a total amount of about 1% to about 50%, for example about 2% to about 25% or about 5% to about 15%. Humectants are present typically in amount of about 5 to about 10% by weight in water, typically, about 30 to about 80% by weight of the dentifrice, more typically about 50 to about 70% by weight.

The base dentifrice may also contain an inorganic or a natural or synthetic thickener or gelling agent. Optionally, one or more thickening agents are optionally present in a total amount of about 0.01% to about 15%, in some embodiments about 0.1% to about 10%, in some embodiments about 0.10 to about 5% by weight, in some embodiments about 0.2% to about 5% by weight and in some embodiments about 0.2 to about 1% by weight. These proportions of thickeners in the dentifrice compositions of the present invention in which the film flakes of the present invention are suspended are sufficient to form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, polyvinylpyrrolidone, carboxyvinyl polymers, cellulosic polymers such as hydroxyethylcellulose, carboxymethylcellulose (carmellose) and salts thereof (e.g., carmellose sodium), natural gums such as karaya, xanthan, gum arabic and tragacanth, colloidal magnesium aluminum silicate, colloidal silica and mixtures thereof.

In various embodiments, an dentifrice composition is provided within a single component or phase. In other embodiments, the composition includes both a first and a second component that are separately maintained. Maintaining the components separately requires only that the components are maintained in such a way as to substantially prevent the interaction of one component of the composition with another component of the composition. Typically, a dual component oral care composition is employed where there are one or more incompatible ingredients included in the composition. For example, if the dentifrice comprises two incompatible active ingredients, it is advantageous to maintain them separately. While the films comprising active ingredients generally provide a degree of separation, there may be some migration of active from the film into the carrier, and vice versa, and as such, in some cases it may desirable to provide an entirely separate phase. The separation of components can be accomplished through any means known or to be discovered in the art and includes chemical, physical, and mechanical means of separation of any combination of these. For example, the first and second incompatible components may be combined but certain components are separately maintained by wrapping or encapsulating one or both in a protective film, coating, capsule, micelle, etc.

The low solubility flavorant is present in the dentifrice base in concentrations of 0.025-10% by weight. Typically, low solubility flavorant is present in the base at a concentration of about 0.05 to about 7.5% based on the total weight. In some embodiments, low solubility flavorant is present in a concentration of about 0.1 to about 5% by weight, in some embodiments, about 0.5 to about 2.5% by weight, in some embodiments, about 0.75 to about 2% by weight, in some embodiments, about 1.0 to about 1.5% by weight.

Typically, to prepare the dentifrice base, water, humectants, e.g. glycerin, sorbitol polyethylene glycol are dispersed in a conventional mixer until the mixture becomes a homogeneous gel phase. Into the gel phase are added other ingredients and mixed until a homogeneous phase is obtained. Thereafter the thickener, any flavor and surfactant ingredients are added and the ingredients mixed at high speed until vacuum of about 20 to 100 mmHg.

In some embodiments, the dentifrice base comprises one or more other components selected from the group consisting of: polyethylene glycol, CMC, sodium saccharin, sodium fluoride, sorbitol (70% solution), purified water, colorant, silica zeodent, cocaamidopropyl betaine and sodium lauryl sulfate.

### Low solubility flavorants

In the present invention menthol is the low solubility flavorant. In addition to menthol, other low solubility flavor ingredients or cooling agents, natural or synthetic, may be incorporated into polymer matrix films using in-situ flavoring of films produced free of low solubility flavorants by adding the films produced free of low solubility flavorants into any toothpaste base which comprises the low solubility flavorants.

The low solubility flavorants may be present in the dentifrice base in an amount sufficient for an effective amount to be transferred from the base into the low solubility flavorant-free poly matrix films within a time period typically about 1 hour to about 7 days after the introduction of such films into the base.

### Other Components

Additional components may also be included in the dentifrice base and/or the polymer matrix films. In some embodiments, one or more additional components are provided in both the dentifrice base and the polymer matrix film. In some embodiments, one or more additional components are provided in the dentifrice base but not the polymer matrix film. In some embodiments, one or more additional components are provided in the polymer matrix film but not in the dentifrice base.

Preferably, the polymer matrix film and/or the dentifrice base optionally comprises one or more of the following additional components: surface active agents, bulking agents, viscosity modifiers, surfactants, thickeners, humectants, diluents, fillers (in addition to those described above), pH modifying agents, plasticizers, fillers, waxes, texture modifiers, oils, flavoring and/or sweetening agents, colorants, dyes, whitening agents, breath freshening agents, abrasives, polishing agents, preservatives, solvents, and mixtures thereof. In embodiments prophylactic and therapeutic agents such as: cetylpyridinium chloride, chlorhexidene, fluoride ion sources, stannous ion sources, tartar control (anticalculus) agents, antimicrobial (e.g., antibacterial) agents, antioxidants, saliva stimulating agents, antiplaque (e.g., plaque disrupting) agents, anti-inflammatory agents, H2 antagonists, desensitizing agents, nutrients, proteins and combinations and mixtures thereof. It is understood that while general attributes of each of the above categories of materials may differ; there may be some common attributes, and any given material may serve multiple purposes within two or more categories of materials.

### Dentifrice Composition comprising Dentifrice Base and Polymer Matrix Films

The film flakes and strips made from the low solubility flavorant-free polymer matrix film are incorporated in the base dentifrice of the present invention, preferably at a concentration of about 0.05 to 1.0% by weight and preferably 0.1 to about 0.5% by weight. The film flakes or strips are generally added to the dentifrice base as a last step, so as to minimize the shear to which the dentifrice ingredients are subjected to during the prior mixing steps.

Initially, the combined compositions comprises low solubility flavorant-free polymer matrix film in the dentifrice base that comprises low solubility flavorant. Over time, the low solubility flavorant transfers from the dentifrice base into the polymer matrix films.

In some embodiments, the film matrix is rupturable during tooth brushing so that one or more additives such as the low solubility flavorant is released when the dentifrice is applied topically to tooth surfaces, the mechanical agitation created during tooth brushing effecting rupture of the film matrix whereby the entrained ingredient is released to the tooth surface. In some embodiments, the complete release is extended such that the flavor experience continues after the oral care procedure is performed.

### SPECIFIC EMBODIMENTS

The invention is further described in the following example. The example is merely illustrative and do not in any way limit the scope of the invention as described and claimed.

A strong cooling signal can be delivered from polymer matrix film which can be manufactured at a much reduced cost by utilizing menthol (or any low solubility flavor ingredient) in the toothpaste base to saturate the polymer matrix film which is manufactured free of the low solubility flavor.

This approach can be extremely cost effective relative to conventional processes in which about 50% of the flavor in the slurry composition used to make (cast and dry) the films is lost in the drying process. As menthol and other flavor ingredients are expensive, a desirable process is to flavor the film in the toothpaste base through reverse migration of the flavor ingredient(s) of choice.

*In-situ* mentholation produced by mixing toothpaste base containing menthol with plain or unflavored films provides advantages over manufacturing and adding mentholated films into toothpaste base. The product produced using *in situ* mentholation provides the same performances as those in which mentholated films are added to the toothpaste base. The technology provides the following advantages:
reduction of formula cost by eliminating menthol loss during the drying process;
reduction of formula cost by elimination of ethanol in the slurry composition;
simplification of the film making process; and
improved safety of the film making environment.

The results of several migration studies of menthol from flavored films indicated that menthol in the film can quickly migrate to toothpaste base and eventually reach equilibrium concentrations in the film depending on the composition.

Toothpaste made using flavorant free films can be used to deliver flavored films which provide a cooling sensation or taste benefit because the flavorant will transfer from the toothpaste base to the flavor free film by reverse migration of menthol.

The mentholated films and menthol free films were made. The compositions of both slurries are listed in Table 1.

**Table 1**

| Ingredient | A. Menthol film slurry (%) | B. Plain film slurry (%) |
|---|---|---|
| DI Water | 62.35 | 67.91 |
| Methocel E5 | 6.25 | 6.25 |
| Methocel E50 | 5.64 | 5.64 |
| ZnO Powder | 18.66 | 19.89 |
| Ethanol | 5.56 | -- |
| Menthol | 1.23 | -- |
| Tween 80 | 0.31 | 0.31 |

The level of menthol in the menthol film made from slurry A was 2.74% (27, 400 PPM) determined by GC-MS. The films made from above slurries were added in toothpaste. The formulations of toothpaste A containing menthol film and toothpaste B containing menthol plus menthol free films are shown in Table 2.

**Table 2**

| Ingredient | A. Toothpaste with menthol films (%) | B. Toothpaste with plain films (%) |
|---|---|---|
| Polyethylene Glycol 600 | 1.00 | 1.00 |
| CMC 500T | 0.55 | 0.55 |
| Sodium Saccharin | 0.35 | 0.35 |
| Sodium Fluoride | 0.32 | 0.32 |
| Sorbitol (70% solution) | 68.00 | 68.00 |
| Purified Water | 9.95 | 9.75 |
| D&C Red No. 30 | 0.01 | 0.01 |
| Silica Zeodent 114 | 8.00 | 8.00 |
| Silica Zeodent 165 | 8.00 | 8.00 |
| Cocaamidopropyl Betaine | 1.25 | 1.25 |
| Sodium Lauryl Sulfate | 1.57 | 1.57 |
| Film | 1.00 | 1.00 |
| Menthol | -- | 0.20 |
| Total | 100.00 | 100.00 |

Both toothpastes were aged at room temperature. The films in toothpaste A and B were isolated from the base at certain time points. The concentration of menthol in isolated film was determined by GC-MS. The results are summarized in Table 3.

**Table 3**

| Aged Time | Menthol level in originally flavored films isolated from toothpaste A (ppm) | Menthol level in originally unflavored films isolated from toothpaste B (ppm) |
|---|---|---|
| 0 Hour | 27352 | -- |
| 4 Hours | 20056 | 1194 |
| 8 Hours | 17392 | 1508 |
| 24 Hours | 6458 | 1842 |
| 48 Hours | 4092 | 1967 |
| 72 Hours | 3650 | 2008 |
| 1 week | 2947 | 2433 |

The results shown in Table 3 shows that menthol concentrations in menthol films and in menthol free films can reach similar levels or concentrations when aged in toothpaste after a certain period of time under room temperature conditions.

## Claims

1. A method of preparing a dentifrice comprising polymer matrix film with low solubility flavorant therein comprising the steps of:
a) forming a polymer matrix film in the substantial absence of low solubility flavorant, wherein said polymer matrix comprises hydroxypropyl methyl cellulose as a water soluble polymer;
b) forming a dentifrice base comprising menthol as a low solubility flavorant;
c) combining the polymer matrix film with the dentifrice base; and
d) maintaining the combined polymer matrix film and dentifrice base for an amount of time suitable for an effective amount of low solubility flavorant to transfer from said dentifrice base to said polymer matrix film,
wherein the combined polymer matrix film and dentifrice base comprises 0,2 wt% menthol, and wherein the combined polymer matrix film and dentifrice base comprises 1 wt% polymer matrix film.

2. The method of claim 1, wherein the polymer matrix film comprises zinc oxide.

3. The method of claim 1 or 2, wherein the polymer matrix film further comprises one or more additional components selected from the group consisting of: diols, surfactants, starches, colorants, dyes, sweeteners, whitening agents, breath freshening agents, abrasives, cationic prophylactic and therapeutic agents, fluoride ion sources, stannous ion sources, tartar control agents, antimicrobial agents, antioxidants, saliva stimulating agents, antiplaque agents, anti-inflammatory agents, H2 antagonists, desensitizing agents, nutrients, and proteins.

4. The method of any of claims 1-3, wherein the dentifrice base further comprises one or more additional components selected from the group consisting of: diols, surfactants, starches, colorants, dyes, sweeteners, whitening agents, breath freshening agents, abrasives, cationic prophylactic and therapeutic agents, fluoride ion sources, stannous ion sources, tartar control agents, antimicrobial agents, antioxidants, saliva stimulating agents, antiplaque agents, anti-inflammatory agents, H2 antagonists, desensitizing agents, nutrients, and proteins.

5. The method of any of claims 1-4 further comprising the steps of:
forming a low solubility flavorant free polymer matrix film by forming a slurry comprising one or more polymers and free of a low solubility flavorant,
dispensing the slurry on a surface wherein the slurry forms a layer of slurry on the surface, and
drying the layer of slurry to produce the low solubility flavorant free polymer matrix film;
wherein, after drying the slurry layer to form the low solubility flavorant free polymer matrix film, the method optionally further comprises the step of cutting or punching the low solubility flavorant free polymer matrix film to form film flakes or strips of low solubility flavorant free polymer matrix film prior to combining the film with the dentifrice base.

6. The method of any of claims 1-5, wherein polymer matrix film is formed in the substantial absence of alcohol.

7. A dentifrice product comprising with low solubility flavorant-free polymer matrix film in a dentifrice base comprising low solubility flavorant produced by the process comprising the steps of:
a) forming a low solubility flavorant-free polymer matrix film in the substantial absence of low solubility flavorant,
wherein the polymer matrix film comprises hydroxypropyl methyl cellulose;
b) forming a dentifrice base comprising menthol as a low solubility flavorant; and
c) combining the polymer matrix film with the dentifrice base,
wherein the combined polymer matrix film and dentifrice base comprises 0,2 wt% menthol, and wherein the combined polymer matrix film and dentifrice base comprises 1 wt% polymer matrix film.

8. The dentifrice product of claim 7 produced by the process further comprising the steps of:
forming the low solubility flavorant free polymer matrix film by forming a slurry comprising a water soluble polymer free of a low solubility flavorant,
dispensing the slurry on a surface wherein the slurry forms a layer of slurry on the surface, and
drying the layer of slurry to produce the low solubility flavorant free polymer matrix film.

## Patentansprüche

1. Verfahren zur Herstellung eines Zahnpflegemittels umfassend Polymermatrixfilm mit einem Geschmacksmittel geringer Löslichkeit, darin umfassend die Schritte von:
a) Bildung eines Polymermatrixfilms im Wesentlichen in Abwesenheit eines Geschmackmittels mit geringer Löslichkeit, wobei die Polymermatrix Hydroxypropylmethylcellulose als ein wasserlösliches Polymer umfasst;
b) Bildung einer Zahnpflegemittelbasis umfassend Menthol als ein Geschmackmittel mit geringer Löslichkeit;
c) Kombinieren des Polymermatrixfilms mit der Zahnpflegemittelbasis; und
d) Halten von kombiniertem Polymermatrixfilm und Zahnpflegemittelbasis über einen ausreichemden Zeitraum, sodass eine wirksame Menge des Geschmacksmittels mit geringer Löslichkeit von der Zahnpflegemittelbasis auf den Polymermatrixfilm übergeht,
wobei die kombinierte Polymermatrixfilm und Zahnpflegemittelbasis 0,2 Gew.-% Menthol umfasst und
wobei die kombinierte Polymermatrixfilm und Zahnpflegemittelbasis 1 Gew.-% Polymermatrixfilm umfasst.

2. Verfahren nach Anspruch 1, wobei der Polymermatrixfilm Zinkoxid umfasst.

3. Verfahren nach Anspruch 1 oder 2, wobei der Polymermatrixfilm weiterhin umfasst ein oder mehrere Zusatzstoffkomponenten ausgewählt aus der Gruppe bestehend aus: Diolen, Tenside, Stärken, Färbemittel, Farbstoffen, Süßungsmittel, Aufhellungsmittel, Atemauffrischungsmitteln, Abrasivstoffe, kationische prophylaktische und therapeutische Mittel, Fluoridionenquellen, Zinnionenquellen, Zahnsteinkontrollmittel, antimikrobielle Mittel, Antioxidantien, Speichel-stimulierende Mittel, Antiplaque-Mittel, Anti-entzündliche Mittel, H2-Antagonisten, Desensibilisierungsmittel, Nährstoffe und Proteine.

4. Verfahren nach irgendeinem der Ansprüche 1 bis 3, worin die Zahnpflegemittelbasis weiterhin umfasst ein oder mehrere Zusatzstoffkomponenten ausgewählt aus der Gruppe bestehend aus Diolen, Tenside, Stärken, Färbemittel, Farbstoffen, Süßungsmittel, Aufhellungsmittel, Atemauffrischungsmitteln, Abrasivstoffe, kationische prophylaktische und therapeutische Mittel, Fluoridionenquellen, Zinnionenquellen, Zahnsteinkontrollmittel, antimikrobielle Mittel, Antioxidantien, Speichel-stimulierende Mittel, Antiplaque-Mittel, Anti-entzündliche Mittel, H2-Antagonisten, Desensibilisierungsmittel, Nährstoffe und Proteine.

5. Verfahren nach irgendeinem der Ansprüche 1 bis 4, weiterhin umfassend die Schritte von:
Bildung eines Polymermatrixfilms, der frei an Geschmacksmitteln mit geringer Löslichkeit ist, durch Bildung einer Aufschlämmung umfassend ein oder mehrere Polymere und frei von einem Geschmacksmittel mit geringer Löslichkeit,
Abgeben der Aufschlämmung auf eine Oberfläche, wobei die Aufschlämmung eine Aufschlämmungsschicht auf der Oberfläche bildet, und
Trocknung der Aufschlämmungsschicht, um einen Polymermatrixfilm, der frei von Geschmacksmitteln mit geringer Löslichkeit ist, zu bilden;
worin nach dem Trocknen der Aufschlämmungsschicht, um einen Polymermatrixfilm, der frei von Geschmacksmittel mit geringem Löslichkeit ist, zu bilden, das Verfahren gegebenenfalls weiterhin umfasst den Schritt des Schneidens oder Stechens des Polymermatrixfilms, der frei von Geschmacksmittel mit geringer Löslichkeit ist, um Filmflocken oder Streifen des Polymermatrixfilms, der frei von Geschmacksmitteln mit geringer Löslichkeit ist, vor dem Kombinieren des Films mit der Zahnpflegemittelbasis zu bilden.

6. Verfahren nach irgendeinem der Ansprüche 1 bis 5, worin der Polymermatrixfilm in Abwesenheit von Alkohol gebildet wird.

7. Zahnpflegemittelprodukt, umfassend einen Polymermatrixfilm, der frei von Geschmacksmitteln mit geringer Löslichkeit ist, in einer Zahnpflegemittelbasis, umfassend Geschmackmitteln mit geringer Löslichkeit, hergestellt durch das Verfahren umfassend die Schritte von:
a) Bildung eines Polymermatrixfilms im Wesentlichen in Abwesenheit eines Geschmackmittels mit geringer Löslichkeit, wobei die Polymermatrix Hydroxypropylmethylcellulose als ein wasserlösliches Polymer umfasst;
b) Bildung einer Zahnpflegemittelbasis umfassend Menthol als ein Geschmackmittel mit geringer Löslichkeit;
c) Kombinieren des Polymermatrixfilms mit der Zahnpflegemittelbasis; und
d) Halten von kombiniertem Polymermatrixfilm und Zahnpflegemittelbasis über einen ausreichemden Zeitraum, sodass eine wirksame Menge des Geschmacksmittels mit geringer Löslichkeit von der Zahnpflegemittelbasis auf den Polymermatrixfilm übergeht,
wobei die kombinierte Polymermatrixfilm und Zahnpflegemittelbasis 0,2 Gew.-% Menthol umfasst und
wobei die kombinierte Polymermatrixfilm und Zahnpflegemittelbasis 1 Gew.-% Polymermatrixfilm umfasst.

8. Zahnpflegemittelprodukt nach Anspruch 7, hergestellt durch das Verfahren weiterhin umfassend die Schritte von:
Bildung eines Polymermatrixfilms, der frei an Geschmacksmitteln mit geringer Löslichkeit ist, durch Bildung einer Aufschlämmung umfassend ein oder mehrere Polymere und frei von einem Geschmacksmittel mit geringer Löslichkeit,
Abgeben der Aufschlämmung auf eine Oberfläche, wobei die Aufschlämmung eine Aufschlämmungsschicht auf der Oberfläche bildet, und
Trocknung der Aufschlämmungsschicht, um einen Polymermatrixfilm, der frei von Geschmacksmitteln mit geringer Löslichkeit ist, zu bilden.

## Revendications

1. Procédé de préparation d'un dentifrice comprenant un film de matrice polymère contenant un agent aromatisant à faible solubilité, comprenant les étapes de :
a) formation d'un film de matrice polymère en l'absence substantielle d'aromatisant à faible solubilité, dans laquelle ladite matrice polymère comprend de l'hydroxypropylméthylcellulose en tant que polymère soluble dans l'eau ;
b) formation d'une base de dentifrice comprenant du menthol en tant qu'aromatisant à faible solubilité ;
c) combinaison du film de matrice polymère avec la base de dentifrice ; et
d) maintien du film de matrice polymère et de la base de dentifrice combinés pendant une durée suffisante pour qu'une quantité efficace d'aromatisant à faible solubilité soit transférée de ladite base de dentifrice audit film de matrice polymère,
dans lequel le film de matrice polymère et la base de dentifrice combinés comprennent 0,2 % en poids de menthol, et
dans lequel le film de matrice polymère et la base de dentifrice combinés comprennent 1 % en poids de film de matrice polymère.

2. Procédé selon la revendication 1, dans lequel le film de matrice polymère comprend de l'oxyde de zinc.

3. Procédé selon la revendication 1 ou 2, dans lequel le film de matrice polymère comprend en outre un ou plusieurs constituants supplémentaires choisis dans le groupe constitué par : les diols, les tensioactifs, les amidons, les colorants, les teintures, les édulcorants, les agents de blanchiment, les agents rafraîchissants l'haleine, les abrasifs, les agents prophylactiques et thérapeutiques cationiques, les sources d'ions fluorures, les sources d'ions stanneux, les agents de lutte contre le tartre, les agents antimicrobiens, les antioxydants, les agents stimulant la salive, les agents antiplaque, les agents anti-inflammatoires, les antagonistes de H2, les agents de désensibilisation, les nutriments et les protéines.

4. Procédé selon l'une quelconque des revendications 1 à 3, dans lequel la base de dentifrice comprend en outre un ou plusieurs constituants supplémentaires choisis dans le groupe constitué par : les diols, les tensioactifs, les amidons, les colorants, les teintures, les édulcorants, les agents de blanchiment, les agents rafraîchissants l'haleine, les abrasifs, les agents prophylactiques et thérapeutiques cationiques, les sources d'ions fluorures, les sources d'ions stanneux, les agents de lutte contre le tartre, les agents antimicrobiens, les antioxydants, les agents stimulant la salive, les agents antiplaque, les agents anti-inflammatoires, les antagonistes de H2, les agents de désensibilisation, les nutriments et les protéines.

5. Procédé selon l'une quelconque des revendications 1 à 4, comprenant en outre les étapes de :
formation d'un film de matrice polymère exempt d'aromatisant à faible solubilité par formation d'une suspension comprenant un ou plusieurs polymères et exempte d'aromatisant à faible solubilité,
distribution de la suspension sur une surface dans laquelle la suspension forme une couche de suspension sur la surface, et
séchage de la couche de suspension pour produire le film de matrice polymère exempt d'aromatisant à faible solubilité ;
dans lequel, après le séchage de la couche de suspension pour former le film de matrice polymère exempt d'aromatisant à faible solubilité, le procédé comprend en outre éventuellement l'étape de découpe ou poinçonnage du film de matrice polymère exempt d'aromatisant à faible solubilité pour former des flocons ou des bandes de film du film de matrice polymère exempt d'aromatisant à faible solubilité avant de combiner le film avec la base de dentifrice.

6. Procédé selon l'une quelconque des revendications 1 à 5, dans lequel un film de matrice polymère est formé en l'absence substantielle d'alcool.

7. Produit dentifrice comprenant un film de matrice polymère exempt d'aromatisant à faible solubilité, dans une base de dentifrice comprenant un aromatisant à faible solubilité produit par le procédé comprenant les étapes de :
a) formation d'un film de matrice polymère exempt d'aromatisant à faible solubilité en l'absence substantielle d'aromatisant à faible solubilité,
dans lequel le film de matrice polymère comprend de l'hydroxypropylméthylcellulose ;
b) formation d'une base de dentifrice comprenant du menthol en tant qu'aromatisant à faible solubilité ; et
c) combinaison du film de matrice polymère avec la base de dentifrice, dans lequel le film de matrice polymère et la base de dentifrice combinés comprennent 0,2 % en poids de menthol, et
dans lequel le film de matrice polymère et la base de dentifrice combinés comprennent 1 % en poids de film de matrice polymère.

8. Produit dentifrice selon la revendication 7, produit par le procédé comprenant en outre les étapes de :
formation du film de matrice polymère exempt d'aromatisant à faible solubilité par formation d'une suspension comprenant un polymère soluble dans l'eau exempt d'aromatisant à faible solubilité,
distribution de la suspension sur une surface dans laquelle la suspension forme une couche de suspension sur la surface, et
séchage de la couche de suspension pour produire le film de matrice polymère exempt d'aromatisant à faible solubilité.
